# EUROPEAN PATENT SPECIFICATION

(11) **EP 0 710 468 B1**
(45) Date of publication and mention of the grant of the patent: **02.01.2002**
(21) Application number: 95307557.9
(22) Date of filing: 24.10.1995
(51) Int. Cl.: A61F 2/12, A61F 2/00

(54) **Method of texturing soft tissue implants using a porous mandrel**
Verfahren zum Texturieren eines weichen Implantats durch Gebrauch einer porösen Form
Procédé pour texturiser un implant souple par l'usage d'un mandrin poreux

(30) Priority: 01.11.1994 US 332877
(43) Date of publication of application: 08.05.1996
(73) Proprietor: MENTOR CORPORATION, Goleta, California 93117 (US)
(72) Inventor: Pai, Suresh, Irving, Texas 75062 (US); Nicosia, Joseph M., Trophy Club, Texas 76262 (US)
(74) Representative: Thomson, Paul Anthony

(56) References cited:
- EP-A- 0 416 846
- FR-A- 2 643 555
- US-A- 3 700 380
- US-A- 4 690 844
- US-A- 4 965 430
- US-A- 5 354 338

## Description

### BACKGROUND OF THE INVENTION

### 1. FIELD OF THE INVENTION :

The present invention is directed to the field of soft tissue implant prostheses, such as breast implants and tissue expanders, and more specifically, is directed to the method of texturing the silicone rubber surface using a porous mandrel as the forming surface for the implant so that the textured surface of the implant impedes capsular contraction and promotes anchoring of the implant. Implants made by the process are also disclosed.

### 2. ART BACKGROUND :

It has been recognized for a number of years that there are substantial advantages in utilizing implant prostheses, such as breast implants, tissue expanders, other tissue implants such as chin and calf implants and the like, which prostheses are formed with a textured exterior surface. A number of benefits of the textured surface have been determined, including a decrease in capsular contraction, increased anchoring strength of the implant which restricts the mobility of the implant, and a decrease in scarring. The advantages of texturing the surface of the implant are described in detail in U.S. Patent Nos. 4,960,425 and 5,022,942 issued to Yan and Purkait

Picha, U.S. Patent Nos. 5,236,453 and 5,002,572 describes a mammary implant in which the surface morphology consists of a plurality of pillar shaped elements.

Hamas, U.S. Patent No. 4,531,244 teaches a mammary prosthesis comprising an envelope covered by a plurality of posts with spaces therebetween, the posts having a depth of about 1,000 to 10,000 microns and a width of the same scope.

Ersek et al., U.S. Patent No. 4,955,909 discloses an implant prosthesis having a surface made up of pillars and indentations of non-uniform dimensions in the range of 20 to 100 microns.

Kitrilakis, U.S. Patent No. 3,700,380 discloses an implant in the form of an artificial vessel, with a textured surface made from embedding dissolvable materials in the surface, curing the surface, and then dissolving the dissolvable materials out of the surface to leave cavities therein.

Banks, U.S. Patent No. 4,329,385 discloses a method of texturing an implant surface, such as a cardiovascular implant by ion beam sputtering of the exterior surface.

EP-A-0416846 discloses a method of manufacturing an envelope for mammary prosthesis intended to implantation, which envelope is provided with a surface having outwardly projecting micropillars integral therewith.

It would be desirable to provide a new method of texturing the surface of an implant which maintains better control over the surface morphology, which at the same time is easier and simpler to apply than prior art processes such as those described above.

### SUMMARY OF THE INVENTION

The present invention is a method of texturing an implant, such as a breast implant, tissue expander, facial implant, or other soft tissue implant, and the implant formed by said method. The method comprises forming a shell on a mandrel, the shell being made of silicone rubber or the like. The depth and porosity of the porous mandrel is specifically selected to form a textured surface while permitting the unobstructed release of the shell from the surface of the mandrel. The porous surface mandrel can be generated from certain porous materials selected from porous ceramic and porous plastic materials to create a textured topography. An example of a suitable porous ceramic includes a porous aluminum oxide. Examples of porous plastics include Teflon® (polytetrafluoroethylene), UHMW polyethylene, and the like.

Preferably, in making the prosthesis shell, a mandrel of the desired shape is used to form the shell, and the mandrel is dipped the required number of times in a silicone rubber dispersion, such as a room-temperature or high temperature vulcanized system, to obtain the desired shell thickness. After the shell is formed it is then cured completely. The shell is then removed from the mandrel and inverted to expose the textured surface.

Once the textured shell is removed from the mandrel, it is assembled with a valve and, if necessary, sealed with a patch and filled with a filler material as is known in the art to form an inflated implant.

There are a number of advantages of this texturing method over the prior art methods. One such advantage is that it is believed that the surface can be more extensively and aggressively textured than with many of the prior art methods. Also, there is substantial control over the uniformity and degree of porosity in the mandrel material so that specifically desired surface morphologies can be achieved. Another advantage of the present invention is that the process can be automated and incorporated into existing processes with minimal required modifications, and therefore, can be developed and used in previously developed industrial manufacturing processes for moderate cost. Another advantage is that the surface is made of a closed cell, insoluble material which is a preferred surface morphology for soft tissue implants. Another advantage is that both the geometry of the texture and the thickness of the implant can be fully controlled during manufacturing, making it a safer and more effective product.

### BRIEF DESCRIPTION OF THE DRAWINGS

**Figure 1** is a partially cutaway illustration of the product produced by the present invention.

**Figure 2** is an enlarged perspective view of the section of the surface of the present invention taken through Figure 1 as designated by the oval section labeled with the numeral 2.

**Figure 3a** is a side view of the mandrel of the present invention.

**Figure 3b** is a bottom view of the mandrel of the present invention.

**Figure 4** is a 40x photomicrograph (SEM-scanning electron microscope) of a section of the surface of the mandrel of the present invention.

**Figure 5** is a 60x photomicrograph (SEM) of a 73° tilt of the surface of the product of the present invention.

**Figure 6** is a 40x plan view photomicrograph (SEM) of the surface of the product of the present invention.

**Figure 7** is a 140x plan view photomicrograph (SEM) of the surface of the product of the present invention.

**Figure 8** is a flowchart of the method of the present invention.

### DETAILED DESCRIPTION OF THE PREFERRED EMBODIMENT

In the process of making the present invention, according to the preferred embodiment, a mandrel suitable for making a prosthesis shell is first selected. The mandrel can be any shape, as is known in the art, depending upon the intended application and desired size. The mandrel is preferably made from a porous ceramic as will be described in more detail below, or a porous plastic, also described below. Other porous materials such as porous metal or porous carbon may also be used.

In the manufacture of a porous ceramic mandrel, the techniques for fabricating porous ceramic substrates are known in the art, and many of the individual techniques are proprietary to the various ceramic manufacturers. The presently preferred ceramic material is commercially available from Coors Ceramics Company (Golden, Colorado) under the designation P100C having pore sizes (approximate diameter) in the range of 87 to 108 microns. The percent apparent porosity which is defined as the pore volume divided by the total volume times 100, of the ceramic material used in the preferred embodiment is 40.2%. However, it will be appreciated by persons of ordinary skill in the art that substantial variations in apparent porosity may be utilized within the scope of the present invention.

In general, a ceramic mandrel as used in the process of the present invention should have a porosity of 300 microns or less, and more preferably, 150 microns or less. Although any method can be used to create the desired porosity, and the specific method of making porous ceramic material is not considered to be a limitation of the present invention. Porous ceramic material is generally made by controlling the lattice structure formation and growth patterns of different crystals contained in the ceramic material, which when fired or cured provide porous voids in the material.
Alternatively, the ceramic material may be doped with a volatile particulate material, such as walnut shell powder, consisting of particles of known size and geometry, which generally disappear when the ceramic is fired, thereby leaving cells or voids.

If porous plastics are used to make the mandrel, the precise techniques are generally considered to be proprietary to the various manufacturers. Porous plastics can be obtained from any of a number of commercial sources including Porex (Fairbanks, Georgia).

The preferred pore size and density depends upon the intended application of the soft tissue implant. Two common types of implants used in plastic surgery are tissue expanders and mammary implants. Tissue expanders are generally indicated for a maximum implantation time of about 6 months during which time they are enlarged in steps over that time. Therefore, more aggressive texturing is desired for these devices, and specifically, the surface should have larger pores and have a greater density of pores than the surface of a mammary implant or other more permanent type of implant. The arrangement and selection of pores minimizes tissue (fibroblast) ingrowth, minimizes mobility of the tissue expander during expansion, reduces the time it takes to complete the expansion regimen, potentially decreases pain, and facilitates removal of the tissue expander. Mammary and other more permanent implants, on the other hand, are implanted over a number of years, and therefore, a more conservative porosity, both in size of the pores and density, are usually required.

After the mandrel is obtained, the silicone rubber polymer for making the shell is selected. A number of appropriate and desirable silicone rubber polymers may be used, and it is contemplated that there are many other materials as well which may be suitable for use in the present invention. Preferably, the polymer is selected from a variety of commercially available room temperature or high temperature vulcanized silicone dispersions (i.e., 30% solids content dispersed in xylene.)

Upon selection and preparation of the raw materials, the implant to be fabricated (in accordance with the present invention) is made by the following steps:

### STEP 1

Insert dipping rod into porous ceramic implant mandrel body. The mandrel may be any desired shape, including round, teardrop, elliptical, crescent, or any other shape). The dipping rod facilitates dipping of mandrel parts into pots of silicone rubber dispersion.

### STEP 1A

Optional: Spray mandrel with a release agent or dip mandrel into a surfactant, such as Du Pont Duponol (30% in 50:50 alcohol:pyrogen free water mixture). The Duponol is applied to minimize residual silicone build up by promoting release of silicone from the mandrel.

### STEP 1B

If Step 1A is performed, dry mandrel in oven at 150°F for 4 to 8 hours to remove all water or carrier (from the release agent) from pores in mandrel.

### STEP 2

Blow any contaminating particulates off of the porous mandrel surface with a destatic air gun.

### STEP 3

Place clean mandrel on dipping racks in dipping canopy to prepare the mandrel for dipping for commercial application.

### STEP 4

Prepare dispersion and adjust the viscosity. The viscosity should be measured and controlled, and in the presently preferred embodiment, is about 40 +/- 10 seconds using a viscometer such as a Zahn Cup. The silicone dispersion is mixed with xylene or some other acceptable diluting solvent to achieve desired viscosity. It is important to control the viscosity of the dispersion in order to control the dipping characteristics and flow rate of dispersion materials. Viscosity control also affects the build rate such that the appropriate thickness of the shell can be produced.

### STEP 5

Remove bubbles from dispersions by placing the dispersion in a vacuum chamber. As is known in the art, air bubble entrapments can compromise the physical integrity of the. implant envelope by introducing point defects in the materials which act as sites of failure.

### STEP 6

Slowly dip mandrel into dispersion as in prior art processes, to prevent splashing, bubbling and uneven build. Care at this step also ensures an even flow and coat.

### STEP 7

Repeat dipping cycle for a minimum of five (5) dips with 10-15 minute devolatilization between dips in a canopy at approximately 32°C (90°F) and at a relative humidity at 35-45%. The dipping protocol in this step has been deemed to be an acceptable dipping procedure to build the desired implant envelope thickness.

### STEP 8

After the last dip, cure the mandrel for 2-6 hours (depending upon the material requirements) at devolatilization conditions 32-43°C (90-110°F) at a relative humidity of 35-45%).

### STEP 9

Remove rod from the mandrel.

### STEP 10

Trim shell around mandrel rod and cut patch hole. A neat hole may then be die cut to facilitate shell removal from the mandrel and the subsequent attachment of valves, patches and the like as is known in the art.

### STEP 11

Remove shell from mandrel and invert inside out, the inside being the texturized surface. The shell is stripped from the mandrel surface by manual lifting and stretching.

### STEP 12

Inspect product and test to ensure that shells meet necessary physical stability and related criteria.

### STEP 13

Stuff shells with Teflon® film to preserve shape during final cure and storage.

### STEP 14

Obtain a patch for the hole in the shell. The patch can be texturized or untexturized. Prepare surface of patch and shell for adhering shell to patch using techniques known in the art for patching a shell. The patch is coated with a layer of unvulcanized or partially vulcanized silicone. The patch with its silicone layer is placed between the patch and the shell so that the patch aligns with the hole in the shell with some overlap between the shell and the patch. The shell and patch assembly are then vulcanized to secure the patch to the shell and thereby form an entire soft tissue implant prosthesis ready for filling.

### STEP 15

Filling can be accomplished by any of a number of methods known in the art. A valve filler system may be employed. Alternatively, injection of filler material into a designated injection site may be used to fill the prosthesis.

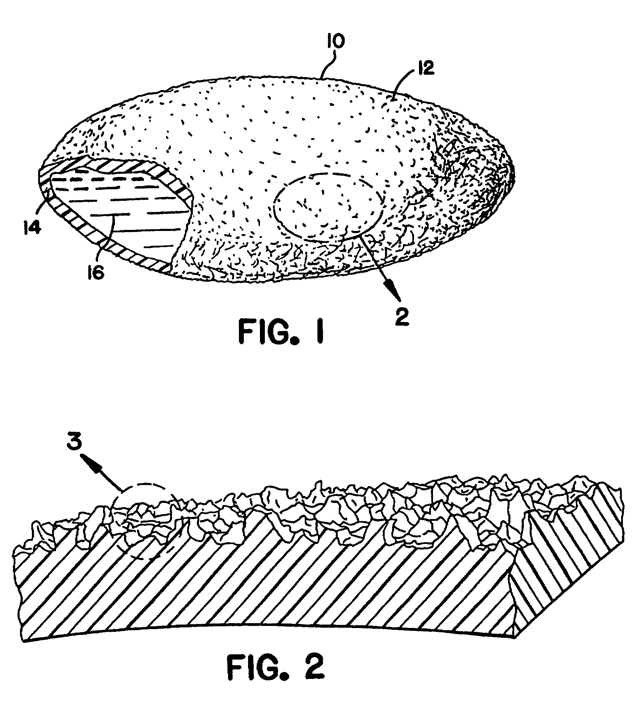

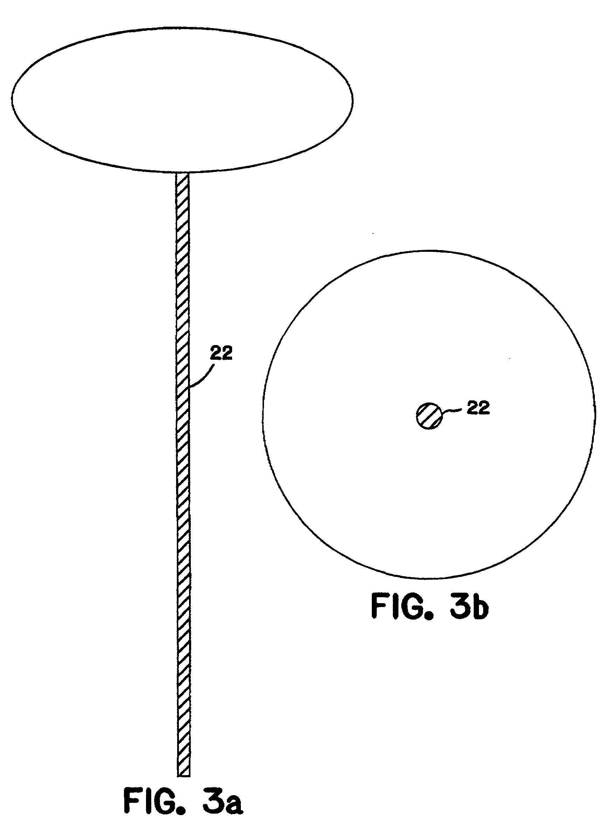

Figures 1-3 show a soft tissue implant texturized in accordance with the present invention. In Figure 1, the implant 10 generally has a texturized exterior surface 12, the texturized surface being formed over a standard wall 14 formed of silicone or the like. As shown in the cutaway section, in use the implant is filled with a filler material 16. As shown in Figure 2, which shows a sectional view of the texturized surface, the surface consists of a multitude of raised, irregularly shaped pores disposed on the surface, the pores being of different sizes. Figures 3a and 3b show the mandrel of the present invention. A handle 22 is attached to the mandrel by any means commonly known in the art, or otherwise. The mandrel may be partially hollow, and may not necessarily be fully enclosed.

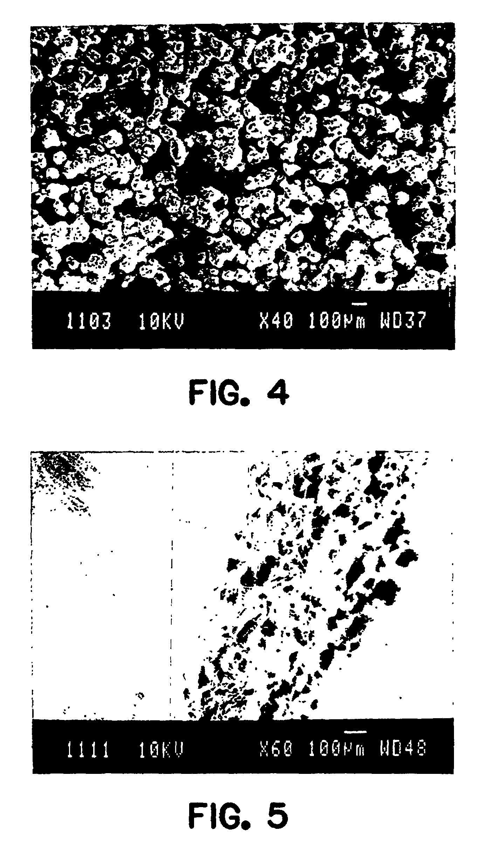

Figure 4 is a scanning electron microscope photomicrograph of a section of the surface of the mandrel of the present invention magnified by 40x.

Figure 5 is a scanning electron microscope photomicrograph of a 73° tilt of the surface of the product of the present invention magnified by 60x.

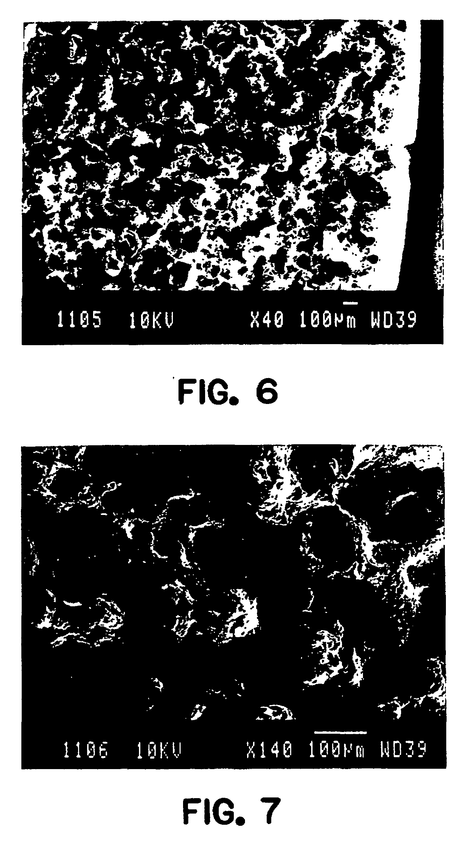

Figure 6 is a scanning electron microscope photomicrograph of a plan view of the surface of the product of the present invention magnified by 40x.

Figure 7 is a scanning electron microscope photomicrograph of a plan view photomicrograph of the surface of the product of the present invention magnified 140×.

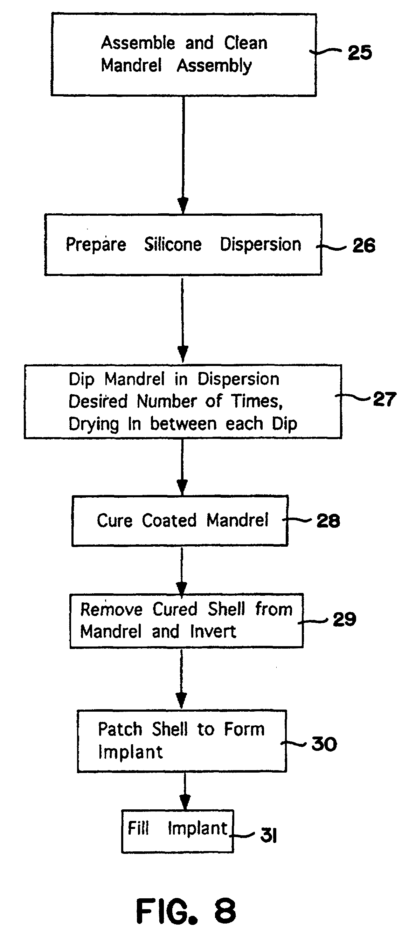

Figure 8 is a flowchart of the method of the present invention. In the first step 25 the surface of the mandrel is cleaned and the mandrel is assembled. In the next step 26, the silicone dispersion is prepared. In the next step 27, the mandrel is dipped in the dispersion a desired number of times, and dried in between each dipping. In the next step 28, the coated mandrel is cured. In the next step 29, the cured shell is removed from the mandrel and inverted, or turned inside out so that the inner surface which had contacted the mandrel becomes the outer surface. In the next step 30, the shell is patched. In the final step 31, the implant is filled.

It will be understood by person of ordinary skill in the art that many changes, additions, deletions and substitutions can be made to the present invention, the presently preferred embodiment of which is described herein, without departing from the scope of the appended claims.

## Claims

1. A method of making a textured soft tissue implant (10) comprising the steps of:
forming a shell (14) on a mandrel in the general shape of the implant (10), the forming step including the steps of
repeatedly coating (27) the mandrel a predetermined number of times with a silicone dispersion (26) to obtain a fully formed shell (14) on the surface of the mandrel;
curing (28) the shell (14);
inverting (29) the cured shell (14) to expose the textured surface (12) of the shell (14) to the outside; and
patching (30) the shell (14) to form the implant (10);
**characterised in that** the mandrel is made of a substantially uniformly porous ceramic, porous plastic, porous metal or porous carbon, said porous material having pore sizes less than 300 microns, the mandrel forming a textured surface (12) on the inside of the shell (14).

2. A method as claimed in claim 1, **characterised in that** the porous material is a porous ceramic.

3. A method as claimed in claim 2, **characterised in that** the porous ceramic has pore sizes of less than 150 microns, preferably in the range of 87 to 108 microns.

4. A method as claimed in claim 2 or 3, **characterised in that** the porous . ceramic has an apparent porosity of about 40%, preferably about 40.2%.

5. A method as claimed in claim 2, 3 or 4, **characterised in that** the porous ceramic is a porous aluminum oxide.

6. A method as claimed in claim 1, **characterised in that** the porous material is a porous plastic.

7. A method as claimed in claim 6, **characterised in that** the porous plastic is polytetrafluoroethylene or UHMW polythene.

8. A method as claimed in claim 1, **characterised in that** the porous material is a porous metal.

9. A method as claimed in claim 1, **characterised in that** the porous material is porous carbon.

10. A method as claimed in any preceding claim, **characterised in that** the repeatedly coating step includes the step of dipping the mandrel in the silicone dispersion at least 5 times, preferably at least 8 times.

11. A method as claimed in any preceding claim, further comprising the step of exposing the coated mandrel to devolatization conditions, preferably including an atmospheric temperature greater than 32°C (90°F) and a relative humidity of at least 35%, and more preferably including an atmospheric temperature ranging from 32 to 43°C (90 - 110°F) and a humidity ranging from 35 to 45%.

12. A method as claimed in any preceding claim, further comprising the step of filling (31) the shell (14) with a filler material (16).

## Patentansprüche

1. Ein Verfahren zur Herstellung eines strukturierten Weichgewebeimplantates (10) mit den Schritten:
ausbilden einer Schale (14) auf einer Form in der allgemeinen Gestalt des Implantates (10), wobei der Ausbildungsschritt folgende Schritte enthält:
wiederholtes beschichten (27) der Form in einer vorbestimmten Anzahl von Schritten mit einer Silikon-Dispersion (26), um eine vollständig ausgebildete Schale (14) an der Oberfläche der Form zu erhalten;
aushärten (28) der Schale (14);
invertieren (29) der ausgehärteten Schale (14), um die strukturierte Oberfläche (12) der Schale (14) nach außen freizulegen; und
abschliessen (30) der Schale (14), um das Implantat (10) zu bilden;
**dadurch gekennzeichnet, daß** die Form aus einer im wesentlichen gleichmäßig porösen Keramik, Kunststoff, Metall oder Kohlenstoff besteht, wobei das poröse Material eine Porengröße von kleiner als 300 µm aufweist, und wobei die Form eine strukturierte Oberfläche (12) auf der Innenseite der Schale (14) bildet.

2. Ein Verfahren nach Anspruch 1, **dadurch gekennzeichnet, daß** das poröse Material eine poröse Keramik ist.

3. Ein Verfahren nach Anspruch 2, **dadurch gekennzeichnet, daß** die poröse Keramik Porengrößen von weniger als 150 µm aufweist, vorzugsweise im Bereich von 87 bis 108 µm.

4. Ein Verfahren nach Anspruch 2 oder 3, **dadurch gekennzeichnet, daß** die poröse Keramik eine wirksame Porosität von etwa 40 %, vorzugsweise etwa 40,2 % aufweist.

5. Ein Verfahren nach den Ansprüchen 2, 3 oder 4, **dadurch gekennzeichnet, daß** die poröse Keramik ein poröses Aluminiumoxid ist.

6. Ein Verfahren nach Anspruch 1, **dadurch gekennzeichnet, daß** das poröse Material ein poröser Kunststoff ist.

7. Ein Verfahren nach Anspruch 6, **dadurch gekennzeichnet, daß** der poröse Kunststoff Polytetrafluoräthylen oder UHMW Polyäthylen ist.

8. Ein Verfahren nach Anspruch 1, **dadurch gekennzeichnet, daß** das poröse Material ein poröses Metall ist.

9. Ein Verfahren nach Anspruch 1, **dadurch gekennzeichnet, daß** das poröse Material poröser Kohlenstoff ist.

10. Ein Verfahren nach einem der vorstehenden Ansprüche, **dadurch gekennzeichnet, daß** der wiederholte Beschichtungsschritt den Schritt beinhaltet, die Form wenigstens fünf mal, vorzugsweise wenigstens acht mal in die Silikon-Dispersion einzutauchen.

11. Ein Verfahren nach einem der vorangegangenen Ansprüche, welches ferner den Schritt aufweist, daß die beschichtete Form Verdunstungsbedingungen ausgesetzt wird, welche vorzugsweise eine Umgebungstemperatur von mehr als 32°C (90°F) und eine relative Luftfeuchte von wenigstens 35 % und, besonders bevorzugt, eine Umgebungstemperatur von 32 bis 43°C (90 bis 110°F) und eine Luftfeuchte von 35 bis 45 % aufweisen.

12. Ein Verfahren nach einem der vorangegangenen Ansprüche, welches ferner den Schritt aufweist, daß die Schale (14) mit einem Füllmaterial (16) gefüllt wird (31).

## Revendications

1. Procédé de fabrication d'un implant souple texturé (10) comprenant les étapes de :
formation d'une coque (14) sur un mandrin (ou forme mère) de la forme générale de l'implant (10), l'étape de formation comportant les étapes de :
enduction (27) du mandrin, répétée un nombre de fois prédéterminé avec une dispersion de silicone (26) pour obtenir une coque (14) pleinement formée sur la surface du mandrin ;
polymérisation (28) de la coque (14) ;
inversion (29) de la coque polymérisée (14) pour exposer la surface texturée (12) de la coque (14) vers l'extérieur ; et
rapiéçage (30) de la coque (14) pour former l'implant (10) ;
***caractérisé en ce que*** le mandrin est fait d'une céramique poreuse, d'une matière plastique poreuse, d'un métal poreux ou de carbone poreux, de porosité sensiblement uniforme, ledit matériau poreux ayant des tailles de pores inférieures à 300 microns, le mandrin formant une surface texturée (12) sur l'intérieur de la coque (14).

2. Procédé selon la Revendication 1, ***caractérisé en ce que*** le matériau poreux est une céramique poreuse.

3. Procédé selon la Revendication 2, ***caractérisé en ce que*** la céramique poreuse a des tailles de pores inférieures à 150 microns, de manière préférée dans la plage de 87 à 108 microns.

4. Procédé selon la Revendication 2 ou 3, ***caractérisé en ce que*** la céramique poreuse a une porosité apparente d'environ 40 %, de manière préférée d'environ 40,2 %.

5. Procédé selon la Revendication 2, 3 ou 4, ***caractérisé en ce que*** la céramique poreuse est un oxyde d'aluminium poreux.

6. Procédé selon la Revendication 1, ***caractérisé en ce que*** le matériau poreux est une matière plastique poreuse.

7. Procédé selon la Revendication 6, ***caractérisé en ce que*** la matière plastique poreuse est le polytétrafluoroéthylène ou du polyéthylène de poids moléculaire extrêmement élevé.

8. Procédé selon la Revendication 1, ***caractérisé en ce que*** le matériau poreux est un métal poreux.

9. Procédé selon la Revendication 1, ***caractérisé en ce que*** le matériau poreux est du carbone poreux.

10. Procédé selon l'une quelconque des Revendications précédentes, ***caractérisé en ce que*** l'étape d'enduction répétée englobe l'étape consistant à plonger le mandrin dans la dispersion de silicone au moins 5 fois, de manière préférée au moins 8 fois.

11. Procédé selon l'une quelconque des Revendications précédentes, comprenant de plus l'étape d'exposition du mandrin enduit à des conditions de dévolatilisation, englobant de manière préférée une température atmosphérique supérieure à 32°C (90°F) et une humidité relative d'au moins 35 %, et de manière davantage préférée, englobant une température atmosphérique comprise dans une plage de 32 à 43°C (90-110°F) et une humidité comprise dans une plage de 35 à 45 %.

12. Procédé selon l'une quelconque des Revendications précédentes, comprenant de plus l'étape de remplissage (31) de la coque (14) avec un matériau de remplissage (16).
